# EUROPÄISCHE PATENTSCHRIFT

(11) **EP 3 758 157 B1**
(45) Veröffentlichungstag und Bekanntmachung des Hinweises auf die Patenterteilung: **25.12.2024**
(21) Anmeldenummer: 19182969.6
(22) Anmeldetag: 27.06.2019
(51) Int. Cl.: H01R 13/40, A61B 18/00, H01R 13/73, H01R 43/00, G06F 13/38, H01R 13/518

(54) **GERÄT MIT SERVICESCHNITTSTELLE UND VERFAHREN ZUR WARTUNG DES GERÄTS**
DEVICE WITH A SERVICE INTERFACE AND METHOD FOR MAINTAINING THE DEVICE
APPAREIL POURVU D'INTERFACE DE SERVICE ET PROCÉDÉ D'ENTRETIEN DE L'APPAREIL

(43) Veröffentlichungstag der Anmeldung: 30.12.2020
(73) Patentinhaber: Erbe Elektromedizin GmbH, 72072 Tübingen (DE)
(72) Erfinder: HILLER, Jürgen, 72581 Dettingen (DE); KEGREISS, Marc, 72108 Rottenburg (DE)
(74) Vertreter: Rüger Abel Patentanwälte PartGmbB

(56) Entgegenhaltungen:
- ES-T3- 2 207 323
- US-A1- 2003 080 630
- US-A1- 2004 214 138
- US-A1- 2005 063 116
- US-A1- 2015 046 125
- US-A1- 2015 229 083

## Beschreibung

Die Erfindung betrifft ein Gerät mit Serviceschnittstelle und ein Verfahren zur Wartung des Geräts, insbesondere unter Sicherstellung einer Zugangskontrolle zu einer Serviceschnittstelle.

Zum Betrieb von medizinischen Instrumenten, insbesondere Kryoinstrumenten, elektrochirurgischen Instrumenten und anderen, insbesondere bei der Operation am menschlichen und tierischen Patienten einzusetzenden Instrumenten, sind Geräte in Gebrauch, die zur Versorgung des betreffenden Instruments mit den benötigten Betriebsmittel eingerichtet sind.

Aus der US 2004/0214138 A1 ist ein Instrument zur Polymerisierung von polymerisierbarem Material bekannt, das beispielsweise im Rahmen von Zahnbehandlungen eingesetzt wird. Das Instrument arbeitet kabellos und enthält eine Lichtquelle, die über eine Batterie versorgt wird. Die Batterie ist über Ladekontakte mit einer Ladestation verbindbar, in welcher das Instrument bei Nichtgebrauch gelagert wird. Das Instrument enthält einen Mikrocontroller, der einerseits mit einem Programm-Wahlschalter verbunden ist und andererseits über eine Datenverbindung mit einem PC kommunizieren kann. Entsprechend sind an dem Instrument Datenübertragungskontakte vorhanden.

Weiter offenbart die US 2015/0229083 A1 ein Gerät zur Versorgung von Instrumenten mit Betriebsmitteln. Dazu sind an der Frontseite des Gerätes entsprechende Steckbuchsen vorgesehen.

Bei dem Betriebsmittel kann es sich um ein stoffliches oder energetisches Mittel handeln. Betriebsmittel können Flüssigkeiten, Gase, Vakuum, elektrische Spannungen, elektrische Ströme, sichtbares oder unsichtbares Licht sein. Das Betriebsmittel kann Leistung und/oder Information tragen und transportieren. Beispiele für Betriebsmittel sind Flüssigkeiten oder Gase wie beispielsweise Wasser, NaCl-Lösung, Argon, Stickstoff, Kohlenstoffdioxyd, Sauerstoff, Aerosole oder dergleichen. Das Betriebsmittel kann dem Instrument zugeführt oder von diesem abgeführt werden (Vakuum), beispielsweise zur Rauchgasabsaugung. Das Betriebsmittel kann Licht, z.B. Laserlicht sein, das dem Instrument zugeführt wird, um eine Wirkung auf biologisches Gewebe auszuüben. Es kann sich auch um Licht zur Operationsfeldbeleuchtung handeln, das dem Instrument zugeführt wird. Auch kann von dem Instrument aufgenommenes Licht zu dem Gerät geleitet werden. Ist das Betriebsmittel elektrischer Strom, kann es sich um Gleichstrom, Wechselstrom, hochfrequenten Wechselstrom handeln, der beispielsweise zum Koagulieren, Thermofusionieren, Abladieren, Schneiden oder zur Bewirkung anderer Effekte dient. Das Gerät kann darauf eingerichtet sein, eins oder mehrere der genannten Betriebsmittel bereitzustellen und/oder aufzunehmen. Beides wird von dem Begriff der "Versorgung" umfasst.

Solche Geräte sind aus dem Stand der Technik wie z. B. aus der oben genannten US 2015/0229083 A1 prinzipiell bekannt. Weiter offenbart dazu die EP 2 904 982 A1 ein Gerät mit einem allseits geschlossenen Gehäuse, das an seiner Front Aufnahmefächer für Buchseneinsätze aufweist. Jeder Buchseneinsatz schließt frontseitig mit der Gerätevorderseite bündig ab und bildet somit aus Sicht des Nutzers einen Teil der Frontfläche des Geräts. Der Buchseneinsatz enthält elektrische Anschlüsse (Buchsen) zum Anschluss eines chirurgischen Instruments. Der Buchseneinsatz ist in dem Aufnahmefach durch ein lösbares Verriegelungsmittel arretiert und an seiner von der Frontseite abgewandten Seite mit dem Gerät über eine Steckverbindung verbunden.

Solche Geräte enthalten normalerweise Steuerungen oder sonstige elektrische Schaltungen, die parametrierbar sind und/oder programmgesteuert sind und/oder im Betrieb Daten gewinnen und speichern und/oder auf Basis von abgespeicherten Daten arbeiten. Zu Service- und Wartungszwecken ist es erforderlich, Wartungstechnikern auf die elektrischen Schaltungen, d.h., deren Parameter und/oder Daten und/oder Programme Zugriff zu gewähren. Dazu ist typischerweise eine Serviceschnittstelle vorhanden, die jedoch elektrisch vom Leistungsteil des Geräts getrennt sein muss, um Schäden für Personen und Material auch bei unbedarftem Umgang auszuschließen.

Davon ausgehend ist es Aufgabe der Erfindung, ein Gerät zur Versorgung von medizinischen Instrumenten zu schaffen, das eine Serviceschnittstelle aufweist, wobei jedoch mit einfachen Mitteln die Schädigung von Benutzern ausgeschlossen werden soll.

Diese Aufgabe wird mit dem Gerät nach Anspruch 1 und/oder dem Verfahren nach Anspruch 13 gelöst. Bevorzugte Ausführungsformen sind in den abhängigen Ansprüchen definiert.

Nach dem erfindungsgemäßen Konzept weist das erfindungsgemäße Gerät eine Serviceschnittstelle in Gestalt eines Datensteckverbinders auf, die bzw. der in dem Aufnahmefach des Buchseneinsatzes angeordnet ist, sodass die Serviceschnittstelle bei betriebsfertig bereitstehendem Gerät von dem Buchseneinsatz verdeckt und somit für den Bediener unzugänglich ist. Für den Wartungstechniker, der Zugang zu dem Datensteckverbinder und somit zur Gerätsteuerung benötigt, ist der Buchseneinsatz aus dem Aufnahmefach herausnehmbar. Typischerweise ist der Buchseneinsatz durch ein Verriegelungsmittel in dem Aufnahmefach gesichert. Das Verriegelungsmittel kann so gestaltet sein, dass es mittels eines Werkzeugs lösbar ist. Beispielsweise kann das Verriegelungsmittel einen Entriegelungsstift enthalten, der in einer von der Frontseite des Buchseneinsatzes zugänglichen Bohrung her axial bewegt werden kann, um dadurch das Verriegelungsmittel zu lösen.

Nach Entnahme des Buchseneinsatzes aus dem Aufnahmefach ist die Serviceschnittstelle zugänglich, die beispielsweise als Datensteckverbinder (männlich oder weiblich) ausgebildet ist. Ist die Serviceschnittstelle beispielsweise ein genormter Steckverbinder, wie z.B. ein USB-Steckverbinder oder dergleichen, kann nun ein entsprechendes Kabel an diesen angeschlossen und somit die Verbindung zu einem externen Gerät, beispielsweise einem Laptop, hergestellt werden. Der Datensteckverbinder ist, wenn der Buchseneinsatz in dem Aufnahmefach angeordnet ist, von dem Buchseneinsatz verdeckt und vorzugsweise nicht mit diesem verbunden, sondern frei. Um dies sicherzustellen, kann der Buchseneinsatz oder jede andere Serviceschnittelle so ausgebildet sein, dass er keine mit dem Datensteckverbinder verbindbaren Anschlüsse aufweist.

An der von der Frontseite abliegenden Begrenzungswand des Aufnahmefachs, d.h., an dessen Rückseite, sind der Betriebsmittelausgang und die Serviceschnittstelle z.B. in Form des Datensteckverbinders angeordnet. Der Betriebsmittelausgang und der Datensteckverbinder sind vorzugsweise zueinander in einem seitlichen Abstand angeordnet, der eine sichere elektrische Isolation zwischen dem Betriebsmittelausgang und dem Datensteckverbinder ermöglicht. Der Datensteckverbinder und der Betriebsmittelausgang sind somit räumlich getrennt. Durch die räumliche Trennung und durch die Abdeckung durch den Buchseneinsatz kann auf elektrische Potentialtrennungsmaßnahmen für den Datensteckverbinder verzichtet werden. Dies senkt den konstruktiven Aufwand und den Platzbedarf, der sonst für eine sichere hochspannungsfeste Trennstrecke in der von dem Datensteckverbinder weg führenden Datenleitung vorgesehen werden müsste.

Typischerweise ist dem Gerät ein Serviceeinsatz zugeordnet, der vom Servicetechniker bereitgehalten und mitgeführt werden kann. Der Serviceeinsatz weist eine Außenkontur auf, die ein Einführen des Serviceeinsatzes in das Aufnahmefach ermöglicht. Der Serviceeinsatz weist ein Gehäuse auf, das an einer Endfläche ein zur Serviceschnittstelle passendes Gegenstück, z.B. einen Datensteckverbinder (weiblich oder männlich) aufweist, der an den Datensteckverbinder des Geräts angepasst ist. Der Datensteckverbinder ist dabei an einer solchen Stelle der Endfläche angeordnet, dass er beim Einführen in das Aufnahmefach mit dem Datensteckverbinder des Geräts in Eingriff kommt und eine korrekte elektrische Verbindung hergestellt ist.

Der Servicesteckverbinder weist typischerweise keinen Anschluss für den Betriebsmittelausgang des Geräts auf, sodass der Serviceeinsatz mit dem Betriebsmittelausgang des Geräts unverbunden ist, und zwar auch dann, wenn der Serviceeinsatz in das Aufnahmefach eingesetzt ist.

Der Serviceeinsatz kann rein passiv ausgebildet sein, indem er an einem in Benutzung aus dem Aufnahmefach herausschauenden Abschnitt seines Gehäuses oder schlicht an seiner Vorderseite eine Steckbuchse, z.B., eine USB-Buchse aufweist. Andere Steckbuchsen wie z.B. zwei- oder mehrpolige Steckbuchsen, Netzwerkbuchsen oder dergleichen können zusätzlich oder alternativ vorgesehen sein. Eine oder mehrere solcher Steckbuchsen bilden eine Kommunikationsschnittstelle, an die ein andere datenverarbeitendes Gerät, d.h. ein Computer, ein Laptop, ein Notebook, ein Tablet, ein Smartphone oder dergleichen angeschlossen werden kann.

Alternativ kann die Schnittstelle zur Kommunikation mit einem anderen Computer als auch Netzwerkschnittstelle oder als Funkverbindung ausgebildet sein. Dazu kann der Serviceeinsatz ein Funkmodul, z.B. ein Bluetooth-Modul oder ein WLAN-Modul enthalten, das zur Kommunikation mit entsprechenden Gegenstellen z.B. in Gestalt eines Computers, eines Laptops, eines Notebooks, eines Tablets, eines Smartphones oder dergleichen eingerichtet ist.

Ergänzend oder alternativ ist es möglich, dass der Serviceeinsatz eigene Speichermittel und/oder eigene Datenverarbeitungsmittel enthält, die durch manuelle Bedienmittel oder über eine Kommunikationsschnittstelle von einem anderen elektronischen Gerät wie z.B. einem mobilen Endgerät (Mobiltelefon, Tablet, Laptop, Notebook, usw.) bedienbar oder aktivierbar ist. Das Speichermittel kann darauf ausgelegt sein, direkt oder über Vermittlung eines in dem Serviceeinsatz untergebrachten Computers Daten oder Programme mit der Steuerung des Geräts auszutauschen.

Das bevorzugte Verfahren zur Wartung eines Geräts umfasst die Entnahme des Buchseneinsatzes aus dem Aufnahmefach, das Einsetzen des Serviceeinsatzes in das Aufnahmefach, die Aktivierung des Serviceeinsatzes, die Entnahme des Serviceeinsatzes aus dem Aufnahmefach und das Wiedereinsetzen des Buchseinsatzes in das Aufnahmefach.

Das Einsetzen des Serviceeinsatzes in das Aufnahmefach umfasst die Kopplung der Datensteckverbinder des Geräts und des Serviceeinsatzes. Das Aktivieren des Serviceeinsatzes umfasst das Freigeben des Datenaustausches zwischen dem Serviceeinsatz und der Gerätesteuerung oder des Datenaustausches zwischen einem an den Serviceeinsatz angeschlossenen datenverarbeitenden Geräts (Computer, Laptop, Notebook, Tablet, Smartphone) und der Gerätesteuerung über den Serviceeinsatz. Es ist möglich, dass der Datenaustausch lediglich das Übertragen von Daten von dem Serviceeinsatz auf die Gerätesteuerung beinhaltet. Es ist auch möglich, dass der Datenaustausch lediglich das Übertragen von Daten von der Gerätesteuerung auf den Serviceeinsatz beinhaltet. Es ist weiterhin auch möglich, dass der Datenaustausch die Datenübertragung in beiden vorgenannten Richtungen umfasst.

Die ausgetauschten Daten können auch oder ausschließlich Anwenderprogramme sein. Die ausgetauschten Daten können Betriebsdaten oder Betriebsparameter des Geräts oder auch vom Gerät gesammelte Messwerte sein, die auf einem Speicher des Geräts oder einem Speicher gehalten werden, der in dem Serviceeinsatz vorgesehen ist. Ergänzend oder alternativ können die Daten über die oben erwähnte Datenschnittstelle an ein externes Gerät, wie beispielsweise ein dem Servicetechniker zur Verfügung stehendes Computergerät wie beispielsweise ein Mobiltelefon, ein Tablet, ein Laptop, ein Notebook oder dergleichen übertragen werden.

Einzelheiten eines exemplarischen Ausführungsbeispiels der Erfindung sind der Zeichnung, den Unteransprüchen oder der zur Zeichnung gehörigen Beschreibung zu entnehmen. Es zeigen:
Figur 1 ein erfindungsgemäßes Gerät in ausschnittsweiser vereinfachter Perspektivdarstellung mit Blickrichtung auf seine Frontseite,
Figur 2 das Gerät nach Figur 1 mit herausgenommenen Buchseneinsatz, in Perspektivdarstellung,
Figur 3 den Buchseneinsatz für das Gerät nach Figur 1 und 2, in perspektivischer Darstellung,
Figur 4 einen Serviceeinsatz für das Gerät nach Figur 1 und 2,
Figur 5 den Buchseneinsatz nach Figur 3, in Rückansicht,
Figur 6 den Serviceeinsatz nach Figur 4, in Rückansicht,
Figur 7 das Gerät nach Figur 1 und 2 in schematischer Blockdarstellung,
Figur 8 eine alternative Ausführungsform des erfindungsgemäßen Geräts in schematischer Blockdarstellung.

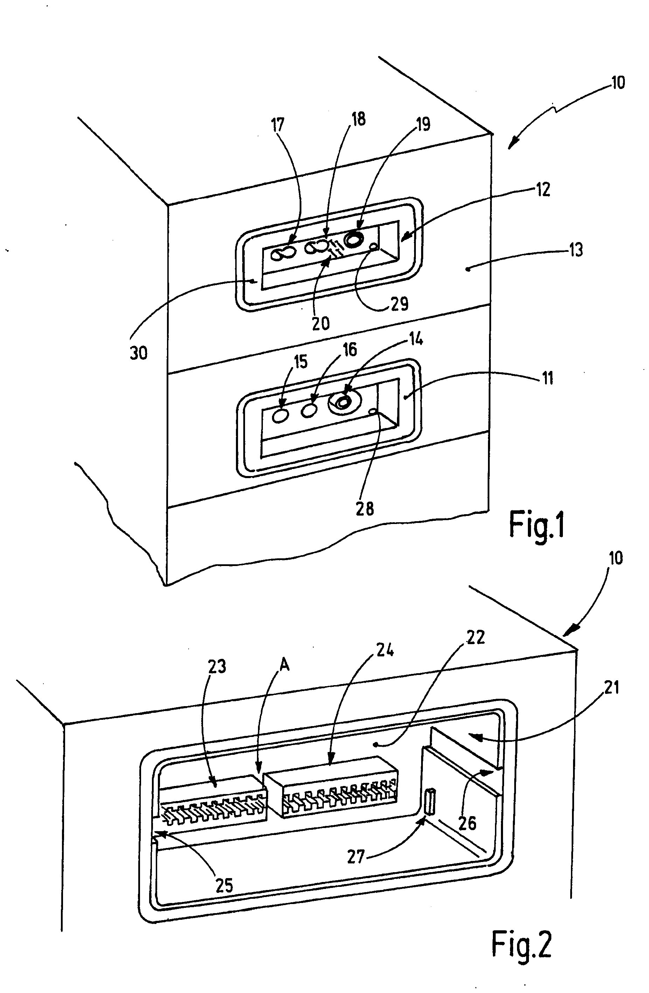

In Figur 1 ist ein Gerät 10 veranschaulicht, das zur Speisung eines oder mehrerer nicht dargestellter Instrumente dient. Das exemplarisch veranschaulichte Gerät 10 dient z.B. zur Versorgung des jeweiligen medizinischen Instruments mit Betriebsmitteln wie beispielsweise fluiden Medien, das heißt zum Beispiel Gasen, Aerosolen und/oder Flüssigkeiten. Der Begriff der "Versorgung" umfasst auch die Aufnahme von Medien aus dem Instrument, das heißt die Bereitstellung von Vakuum oder Absaugleistung zu Absaugzwecken. Das Gerät kann alternativ oder zusätzlich zur Versorgung von einem oder mehreren medizinischen Instrumenten mit elektrischem Strom eingerichtet sein. Zusätzlich oder alternativ kann das Gerät 10 zur Versorgung des medizinischen Instruments mit Licht oder auch zur Aufnahme von Licht eingerichtet sein, das von dem Instrument aufgenommen oder erzeugt worden ist. Auch dies soll unter dem Begriff der "Bereitstellung von Betriebsmitteln" verstanden werden.

Zur Bereitstellung von mindestens einem Betriebsmittel können an dem Gerät 10 ein oder mehrere Anschlussmöglichkeiten für medizinische Instrumente vorgesehen sein. Bei dem in Figur 1 exemplarisch veranschaulichten Gerät sind zwei Anschlussmöglichkeiten gegeben, die durch Buchseneinsätze 11, 12 gegeben sind. Diese Buchseneinsätze 11, 12 sind an der Frontseite 13 des Gerät 10 angeordnet. Die Buchseneinsätze 11, 12 sind vorzugsweise so ausgebildet, dass sie zum Betrieb eines Instruments erforderliche Anschlüsse enthalten. Beispielsweise weist der Buchseneinsatz 11 eine Steckbuchse 14 für ein Fluid und/oder weitere Steckbuchsen 15, 16 für elektrischen Strom auf. Die Steckbuchsen 15, 16 können Betriebsstrom, steuernden Strom um zum Beispiel das Gerät 10 von dem angeschlossenen Instrument auszusteuern oder Behandlungsströme, Messströme und dergleichen führen. Das Gerät kann (muss aber nicht) zusätzliche Anschlüsse enthalten, z.B. einen oder mehrere Anschlüsse für eine Neutralelektrode.

Der Buchseneinsatz 12 ist exemplarisch als rein elektrische Buchse veranschaulicht, die mehrere Steckbuchsen 17, 18, 19 für den Behandlungsstrom eines anzuschließenden Instruments aufweist. Außerdem können an dem Buchseneinsatz 12 ein oder mehrere Pins 20 vorgesehen sein, die zur Erfassung des Typs des eingesteckten Steckers dienen können und die axial fest oder axial beweglich angeordnet sein können. Weiter können ein oder mehrere Anschlüsse vorgesehen sein, die zu Steuerleitungen gehören und über die am Instrument vorgesehene Bedienelemente mit der Gerätesteuerung verbindbar sind.

Die Buchseneinsätze 11, 12 sind lediglich exemplarischer Natur. Es kann auch ein Buchseneinsatz vorgesehen werden, der ausschließlich ein oder mehrere Fluidbuchsen umfasst. Es kann ein Buchseneinsatz vorgesehen sein, der ausschließlich ein oder mehrere Anschlüssen für Licht aufweist, um Licht zu dem Instrument zuleiten oder von diesem aufzunehmen. Außerdem kann ein Buchseneinsatz vorgesehen sein, der ausschließlich ein oder mehrere Anschlüsse aufweist, an denen eine Absaugfunktion (Vakuum) bereitgestellt wird. Es kann ein Buchseneinsatz vorgesehen sein, der mindestens einen Anschluss für Behandlungsstrom, Signalstrom oder sonstige Träger von Energie und/oder Information aufweist. Es ist auch möglich, einen Buchseneinsatz vorzusehen, der zwei oder mehrere der vorgenannten verschiedenartigen Anschlüsse aufweist.

Jeder Buchseneinsatz 11, 12 ist in einem Aufnahmefach angeordnet. Figur 2 veranschaulicht dazu exemplarisch ein Aufnahmefach 21. Das Aufnahmefach 21 weist an seiner Rückwand 22 einen Betriebsmittelausgang 23 und eine Datenschnittstelle z.B. in Gestalt eines Datensteckverbinders 24 auf. Der Betriebsmittelausgang 23 und der Datensteckverbinder 24 sind dabei in seitlichem Abstand A zueinander angeordnet, der eine elektrische Isolation zwischen dem Betriebsmittelausgang 23 und dem Datensteckverbinder 24 und somit die nötigen Luft- und Kriechstrecken erbringt.

Der Betriebsmittelausgang 23 ist als elektrischer Steckverbinder mit vielen Kontakten dargestellt, wobei jedoch der Betriebsmittelausgang nicht auf derartige Steckverbinder beschränkt verstanden werden darf. Der Betriebsmittelausgang 23 kann durch jedes Verbindungsmittel gebildet werden, das zur sicheren Übertragung des jeweiligen Betriebsmittels von dem Gerät 10 auf den Buchseneinsatz 12 (oder 11) geeignet ist. Gleiches gilt für den Datensteckverbinder 24. Auch dieser ist in Figur 2 als vielpolige Steckbuchse dargestellt. Es kann sich bei dem Datensteckverbinder 24 jedoch auch in jedem anderen zur Datenübertragung geeigneten Verbinder handeln.

In dem Aufnahmefach 21 können, falls gewünscht, Führungen 25, 26 für den Buchseneinsatz 12 und/oder zu einer Verriegelungseinrichtung gehörende Rastvorsprünge 27 oder sonstige Rastmittel angeordnet sein, um den Buchseneinsatz 11 oder 12 zum Beispiel in der in der EP 2904982 A1 dargestellten Art zu verriegeln. Entsprechende Lösestifte 28, 29 zum Lösen einer entsprechenden Verriegelung können in den Buchseneinsätzen 11, 12 vorgesehen und von der Frontseite her zugänglich sein.

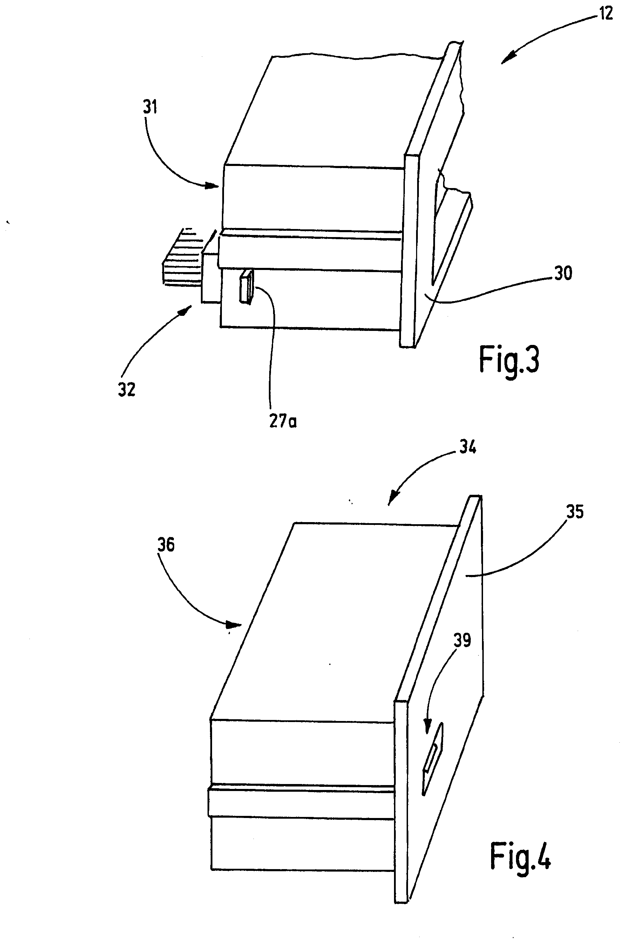

Figur 3 veranschaulicht den Buchseinsatz 12 gesondert in ausschnittsweiser perspektivischer Darstellung. An seiner von der Frontseite 30 abliegenden Rückseite 31 ist ein Betriebsmittelverbinder 32 angeordnet, der zum Beispiel als zu dem Betriebsmittelausgang 23 passender Stecker ausgeführt ist. Der Betriebsmittelverbinder 32 kann eine Anzahl von elektrischen Kontakten aufweisen, die mit Kontakten des Betriebsmittelausgangs 23 in elektrische Führung geraten, wenn der Buchseneinsatz 12 in das Aufnahmefach 21 vollständig eingeschoben und darin verriegelt ist. Die elektrischen Kontakte können mit Kontakten in den Steckbuchsen 17, 18, 19 und/oder mit den Pins 20 verbunden sein. Sollen sonstige Betriebsmittel, wie z.B. Fluid und/oder Licht und/oder Vakuum übertragen werden, hat der Buchseneinsatz an seiner Rückseite 31 und an seiner Frontseite 35 entsprechende Verbinder.

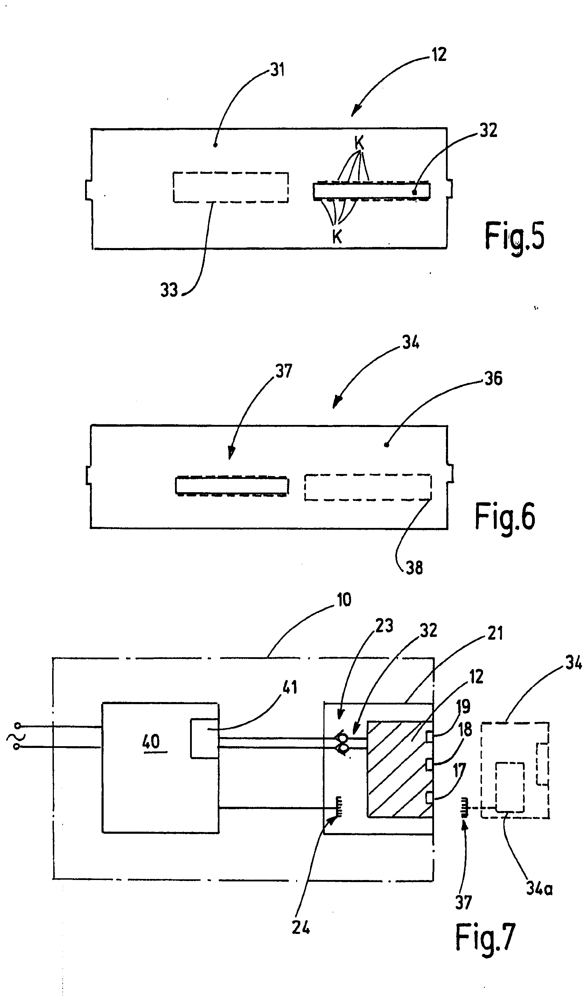

Figur 5 veranschaulicht die Rückseite 31 des Buchseneinsatzes 12 und den Betriebsmittelverbinder 32 mit seinen Kontakten K in Draufsicht. Wie ersichtlich, ist der Betriebsmittelverbinder 32 räumlich und funktional dem Betriebsmittelausgang 23 zugeordnet. Hingegen ist die dem Datensteckverbinder 24 gegenüberliegende Fläche 33, die in Figur 5 zur Veranschaulichung gestrichelt umgrenzt ist, frei.

Im Betrieb stellt der Buchseneinsatz 12 in den Steckbuchsen 17, 18, 19 sowie gegebenenfalls an den Pins 20 die für den Anschluss des Instruments erforderlichen Betriebsströme und/oder Signalströme bereit. Jedoch verdeckt der Buchseneinsatz 12 den Datensteckverbinder 24, so dass dieser für den Benutzer des Geräts 10 und den Benutzer des Instruments unzugänglich bleibt. Das Gerät 10 bietet somit für den nicht autorisierten und nicht eingeweihten Bediener keine Möglichkeit, absichtlich oder unbeabsichtigter Weise an den Datensteckverbinder 24 und somit an interne Einstellungsmöglichkeiten des Geräts 10 zu gelangen.

Dem Servicetechniker steht der Serviceeinsatz 34 zur Verfügung, wie er in den Figuren 4 und 6 veranschaulicht ist. Dieser weist eine Außenkontur auf, die an die Innenkontur des Aufnahmefachs 21 angepasst ist oder zumindest so ausgebildet ist, dass ein Einführen des Serviceeinsatzes 34 in das Aufnahmefach 21 möglich ist. Der Serviceeinsatz 34 kann dabei eine solche Länge aufweisen, dass er mit seiner Frontplatte 35 bündig mit der Frontseite 13 des Geräts 10 abschließt. Alternativ kann der Serviceeinsatz 34 jedoch auch länger ausgebildet sein, sodass er nach dem Einsetzen in das Aufnahmefach 12 aus diesem herausragt. Der Serviceeinsatz 34 kann bedarfsweise mit Verriegelungsmitteln versehen sein, um ihn in dem Aufnahmefach 21 zu arretieren. Alternativ können solche Arretierungsmittel auch fehlen.

Der Serviceeinsatz 34 weist an seiner an der Frontplatte 35 wegweisenden Rückseite 36 einen Datensteckverbinder 37 (oder eine sonstige mit der Datenschnittstelle des Geräts 10 in Verbindung bringbare Einrichtung) auf, dessen Form und Position an den Datensteckverbinder 24 des Aufnahmefachs 21 angepasst ist. Der Datensteckverbinder 37 kann als mehrpoliger elektrischer Steckverbinder ausgebildet sein, der eine Anzahl von Kontakten aufweist, die mit Kontakten des Datensteckverbinders 24 in Kontakt kommen, wobei der Serviceeinsatz 34 in das Aufnahmefach 21 eingesetzt ist. Der dem Betriebsmittelausgang 23 dabei gegenüber liegende Teil 38 der Rückseite 36 ist in Figur 6 durch eine gestrichelte Linie umgrenzt dargestellt. Diese Fläche, d.h., dieser Teil 38 der Rückseite 36 ist frei, d.h., dort sind keine Elemente angeordnet, die mit Kontakten des Betriebsmittelausgangs 23 in elektrischen Kontakt kommen könnten.

Der Serviceeinsatz 24 kann im einfachsten Fall als passives Element ausgebildet sein, indem an seiner Frontplatte 35 eine Steckbuchse 39 zum Anschluss eines elektrischen datenverarbeitenden Geräts vorgesehen ist. Die Kontakte der Steckbuchse 39 können mit entsprechenden Kontakten des Datensteckverbinders 37 verbunden sein. Die Steckbuchse 39 kann eine beliebige Steckbuchse, zum Beispiel eine USB-Buchse, eine Netzwerkbuchse oder dergleichen sein.

Die Benutzung und Wartung des Geräts 10 ist wie folgt:
Bei normalem Betrieb ist der Buchseneinsatz 12 für den Anschluss eines Instruments vorgesehen und betriebsfähig. Soll das Gerät 10 zum Betrieb anderweitiger Instrumente hergerichtet werden, kann dies vom Wartungstechniker durch Wechsel des Buchseneinsatzes 12 ermöglicht werden, indem dieser den Buchseneinsatz 12 durch einen anderen geeigneten Buchseneinsatz austauscht.

Will der Wartungstechniker hingegen die Software des Geräts 10 aktualisieren, das Gerät 10 abweichend von der bisherigen Einstellung parametrieren, Betriebsdaten oder Messwerte aus dem Gerät 10 auslesen oder sonstige Daten ein- oder auslesen, benötigt er Zugang zur Serviceschnittstelle des Geräts 10. Diese wird durch den Datensteckverbinder 24 gebildet. Zu diesem Zweck entnimmt der Servicetechniker dem Gerät zunächst den Buchseneinsatz 12 und hat nun Zugang zu dem Datensteckverbinder 24. Ist dieser (anders als in Figur 2 dargestellt) als Netzwerkbuchse oder als USB-Buchse ausgebildet kann er seinen Laptop, sein Notebook oder ein anderes Datenverarbeitendes Gerät direkt an diese Buchse anschließen. Bevorzugt wird aber in dem Gerät 10 ein Datensteckverbinder 24 vorgesehen, der nicht in Normen einer Netzwerksteckdose oder einer USB-Steckdose entspricht, sodass ein unbefugter Zugriff auf die Gerätesoftware weiter erschwert wird. Dem Servicetechniker steht jedoch der Serviceeinsatz 34 zur Verfügung, den er nun in das Aufnahmefach 12 einsetzt. Ist dies geschehen, kann er an der Frontplatte 35 bzw. der Steckbuchse 39 ein Kabel anschließen, um eine Verbindung zwischen seinem datenverarbeitenden Gerät (Laptop, Notebook, usw.) und der Steuerung 40 des Geräts 10 herstellen (siehe Figur 7).

Nach durchgeführter Wartung entfernt der Servicetechniker den Serviceeinsatz 34 aus dem Aufnahmefach 21 und setzt den Buchseneinsatz 12 (oder falls ein Wechsel gewünscht ist einen anderen Buchseneinsatz) in das Aufnahmefach 21 ein.

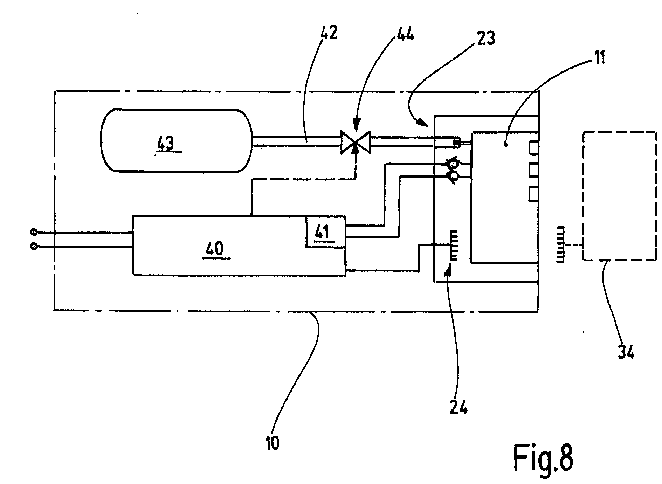

Während Figur 7 schematisch ein Gerät 10 mit einem elektrischen Leistungsteil 41 veranschaulicht, der den Betriebsmittelausgang 23, beispielsweise mit hochfrequenter Wechselspannung speist, die zum Koagulieren, Abladieren oder Schneiden vom biologischem Gewebe geeignet ist, zeigt Figur 8 ein Gerät 10, dessen Steuerung 40 zusätzlich dazu einen Fluidkanal 42 kontrolliert. Dieser führt von einer Gasquelle 43, die als externe Gasquelle oder auch wie dargestellt innerhalb des Geräts 10 ausgebildet sein kann, über mindestens ein Ventil 44 zu dem Betriebsmittelausgang 23. Der Betriebsmittelausgang 23 umfasst hier einen Steckverbinder für Fluide sowie Steckverbinder für die elektrische von dem Leistungsteil 41 abgegebene Spannung bzw. den Strom.

Eine solche Konfiguration kann zur Speisung des Buchseneinsatzes 11 dienen wie er in Figur 4 veranschaulicht ist. Wiederum kann der Buchseneinsatz 11 entnommen und zu Wartungszwecken durch den Serviceeinsatz 34 ersetzt werden, der dann den Servicetechniker Zugang zu der Steuerung 40 bietet.

Anstelle eines Buchseneinsatzes 11, der sowohl fluide Medien als auch Strom überträgt, kann auch ein Buchseneinsatz 11 vorgesehen sein, der nur fluide Medien und/oder andere Leistungs- oder Informationstragende Medien, wie zum Beispiel Licht überträgt. Auch kann der Fluidkanal 42 als Absaugkanal ausgebildet sein.

Bei allen vorstehend beschriebenen Ausführungsformen kann anstelle eines passiven Serviceeinsatzes 34, wie er in Figur 4 dargestellt ist, ein Serviceeinsatz vorgesehen sein, der selbst ein Speichermedium und/oder wenigstens eine informationsverarbeitendes Element, wie beispielsweise einen Mikrocontroller enthält. Ein solcher Serviceeinsatz 34 kann beispielsweise darauf eingerichtet sein, Daten oder Programme in die Steuerung 40 einzuspielen, nachdem der Serviceeinsatz 34 in das Aufnahmefach 21 eingesetzt ist. In diesem Fall kann die Frontplatte 35 zum Beispiel ohne Verbindungsmöglichkeiten und Anschlussmöglichkeiten für einen weiteren Rechner ausgebildet sein. Ein solcher Serviceeinsatz 34 kann zusätzlich oder alternativ auch darauf eingerichtet sein, Daten aus der Steuerung 40 auszulesen und in sich abzuspeichern.

An einem solchen Serviceeinsatz 34 kann auch die in Figur 4 veranschaulichte Steckbuchse vorgesehen sein, um ein externes Gerät, wie beispielsweise einen Laptop oder dergleichen, anzuschließen, um eine Datenübertragung von dem Serviceeinsatz 34 auf die Steuerung 40 oder von der Steuerung 40 auf den Serviceeinsatz 34 zu kontrollieren, zu initiieren oder auf sonstige Weise zu beeinflussen.

Der Serviceeinsatz 34 kann auch ein Funkmodul, beispielsweise ein Bluetoothmodul oder ein WLAN-Modul enthalten, um eine Verbindung zu einem externen datenverarbeitenden Gerät, wie beispielsweise einem Mobiltelefon, einem Tablet, einem Notebook oder einem Laptop herzustellen, um somit eine Kommunikation zwischen der Steuerung 40 und dem jeweiligen mobilen Endgerät zu ermöglichen. Dabei kann der Serviceeinsatz 34 zusätzlich zu dem Funkmodul Speichermittel und/oder datenverarbeitende Mittel enthalten.

Das erfindungsgemäße Gerät 10 weist einen Buchseneinsatz 11 auf, der in einem Aufnahmefach 21 des Geräts 10 angeordnet ist. Der Buchseneinsatz 11 ist dabei an eine Betriebsmittelschnittstelle 34 des Geräts angeschlossen. In dem Aufnahmefach 21 ist zusätzlich eine Datenschnittstelle 24 angeordnet, die von dem Buchseneinsatz 11 verdeckt und damit von außen unzugänglich ist.

Um Zugriff auf die Gerätesoftware und/oder Gerätedaten zu erhalten, kann der Buchseneinsatz 11 entfernt und durch einen Serviceeinsatz 34 ersetzt werden, der einerseits den Betriebsmittelausgang 23 verdeckt dafür aber den Datensteckverbinder 24 kontaktiert. In diesem Zustand kann der Serviceeinsatz 34 direkt oder ein externes Gerät (Laptop, Notebook, Tablet, usw.) vermittels des Serviceeinsatzes 34 mit der Gerätesteuerung 40 kommunizieren, um Daten und/oder Programme ein- oder auszulesen sowie Programme zu aktualisieren, Daten zu ändern, um Geräteparameter zu ändern oder dergleichen. Die durch den Buchseneinsatz 11, 12 verdeckte Anordnung der Serviceschnittstelle stellt ein wirksames Mittel zur Zugangskontrolle zu der Serviceschnittstelle dar. Es erschwert oder verhindert unbefugten Zugriff auf die Schnittstelle und Schäden für Personen und Material, die sonst auf Grund der fehlenden durchschlagsicheren Potentialtrennung zwischen der Serviceschnittstelle und insbesondere dem Leistungsteil der Gerätesteuerung auftreten könnten.

### Bezugszeichen:

- 10: Gerät
- 11: Buchseneinsatz
- 12: Buchseneinsatz
- 13: Frontseite
- 14: Steckbuchse für Fluid oder für bipolaren Koaxialstecker - je nach Ausführungsform
- 15 - 19: Steckbuchsen für elektrischen Strom
- 20: Pins
- 21: Aufnahmefach
- 22: Rückseite
- 23: Betriebsmittelausgang
- 24: Datensteckverbinder
- A: Abstand
- 25, 26: Führungen
- 27: Rastvorsprung - Verriegelungsvorrichtung 27a
- 28, 29: Lösestifte
- 30: Frontseite
- 31: Rückseite
- 32: Betriebsmittelverbinder
- K: Kontakte
- 33: freie Fläche
- 34: Serviceeinsatz
- 35: Frontplatte
- 36: Rückseite
- 37: Datensteckverbinder
- 38: Bereich/Fläche
- 39: Steckbuchse
- 40: Steuerung
- 41: Leistungsteil
- 42: Fluidkanal
- 43: Gasquelle
- 44: Ventil

## Patentansprüche

1. Gerät (10), insbesondere zur Versorgung von medizinischen Instrumenten mit Betriebsmittel, insbesondere einem oder mehreren fluiden Medien und/oder elektrischem Strom,
mit einem Gehäuse, in dem eine elektrische Schaltung (40) angeordnet ist, die mindestens einen Betriebsmittelausgang (23) aufweist oder mit einem solchen steuernd verbunden ist und die mindestens einen Datensteckverbinder (24) aufweist,
mit einem Aufnahmefach (21), in dem der Betriebsmittelausgang (23) und der Datensteckverbinder (24) angeordnet sind,
mit einem Buchseneinsatz (11, 12), der den Datensteckverbinder (24) abdeckend in dem Aufnahmefach (21) angeordnet ist und der geräteseitig einen Betriebsmittelverbinder (32) aufweist, der mit dem Betriebsmittelausgang (23) lösbar verbunden ist und der einen von außen her zugänglichen Instrumentenanschluss (14 - 19) aufweist, der mit dem Betriebsmittelverbinder (32) verbunden ist, wobei der Buchseneinsatz (11, 12) mit dem Datensteckverbinder (24) unverbunden ist und keine mit dem Datensteckverbinder (24) verbindbaren Anschlüsse aufweist.

2. Gerät nach **Anspruch 1,** wobei der Datensteckverbinder (24) und
der Betriebsmittelausgang (23) in einem Abstand zueinander in dem Aufnahmefach (21) angeordnet sind.

3. Gerät nach einem der vorstehenden Ansprüche, wobei dem Buchseneinsatz (11, 12) eine Verriegelungsvorrichtung (27a) zur Arretierung des Buchseneinsatzes (11, 12) in dem Aufnahmefach (21) zugeordnet ist.

4. Gerät nach einem der vorstehenden Ansprüche, wobei der Buchseneinsatz (11, 12) eine dem Aufnahmefach (21) angepasste Kontur aufweist.

5. Gerät (10) nach einem der vorstehenden Ansprüche, mit einem Serviceeinsatz (34), der Gehäuse umfasst, das an einer Endfläche (36) einen Datensteckverbinder (37) aufweist, der an den Datensteckverbinder (24) des Geräts (10) angepasst ist.

6. Gerät nach Anspruch **5,** wobei der Serviceeinsatz (34) eine an das Aufnahmefach (21) angepasste Kontur aufweist.

7. Gerät nach einem der Ansprüche 5 oder 6 , wobei der Serviceeinsatz (34) frei von Anschlussmitteln für den Betriebsmittelausgang (23) ist.

8. Gerät nach einem der Ansprüche 5 bis 7, wobei der Serviceeinsatz (34) ein Speichermedium (34a) aufweist, das mit dem Datensteckverbinder (37) verbunden ist.

9. Gerät nach einem der Ansprüche 5 bis 8, wobei der Serviceeinsatz (34) eine Schnittstelle (39) zur Kommunikation mit einem Computer aufweist.

10. Gerät nach Anspruch 9, wobei die Schnittstelle (39) eine USB-Schnittstelle ist.

11. Gerät nach Anspruch 9, wobei die Schnittstelle (39) eine Netzwerkschnittstelle ist.

12. Gerät nach Anspruch 9, wobei die Schnittstelle eine Funkverbindung ist.

13. Verfahren zur Wartung eines Geräts nach einem der Ansprüche 5 bis 12 mit folgenden Schritten:
Entnahme des den Datensteckverbinder (24) nicht kontaktierenden Buchseneinsatzes (11, 12) aus dem Aufnahmefach (21),
Einsetzen des den Datensteckverbinder (24) kontaktierenden Serviceeinsatzes (34) in das Aufnahmefach (21), Aktivierung des Serviceeinsatzes (34),
Entnahme des den Datensteckverbinder (24) kontaktierenden Serviceeinsatzes (34) aus dem Aufnahmefach (21),
Einsetzen des den Datensteckverbinder (24) nicht kontaktierenden Buchseneinsatzes (11, 12) in das Aufnahmefach (21).

## Claims

1. Apparatus (10) particularly for supply of medical instruments with operating media, particularly one or more fluidical media and/or electrical current,
having a housing in which an electrical circuit (40) is arranged that comprises at least one operating medium output (23) or is connected with such an operating medium output (23) in a controlling manner and that comprises at least one data plug (24),
having a receptacle (21) in which the operating medium output (23) and the data plug (24) are arranged,
having a socket insert (11, 12) that is arranged in the receptacle (21) covering the data plug (24) and that comprises an operating medium connector (32) on the apparatus side that is releasably connected with the operating medium output (23) and that comprises an instrument connection (14-19) accessible from outside that is connected with the operating medium connector (32), wherein the socket insert (11, 12) is non-connected with the data plug (24) and does not comprise connections connectable with the data plug (24).

2. Apparatus according to claim 1, wherein the data plug (24) and the operating medium output (23) are arranged with distance to each other in the receptacle (21).

3. Apparatus according to any of the preceding claims, wherein a locking device (27a) for locking of the socket insert (11, 12) in the receptacle (21) is assigned to the socket insert (11, 12).

4. Apparatus according to any of the preceding claims, wherein the socket insert (11, 12) has a contour adapted to the receptacle (21).

5. Apparatus (10) according to any of the preceding claims, having a service insert (34) that comprises a housing, which comprises a data plug (37) at an end face (36) that is adapted to the data plug (24) of the apparatus (10).

6. Apparatus according to claim 5, wherein the service insert (34) it comprises a contour adapted to the receptacle (21).

7. Service insert according to any of the claim 5 or 6, wherein the service insert (34) is free from connection means for the operating medium output (23).

8. Service insert according to any of the claims 5 to 7, wherein the service insert (34) comprises a storage medium (34a) that is connected with the data plug (37).

9. Service insert according to any of the claims 5 to 8, wherein the service insert (34) comprises an interface (39) for communication with a computer.

10. Service insert according to claim 9, wherein the interface (39) is a USB-interface.

11. Service insert according to claim 9, wherein the interface (39) is a network interface.

12. Service insert according to claim 9, wherein the interface is a radio transmission.

13. Method for servicing an apparatus according to any of the claims 5 to 12, comprising the following steps:
Removing the socket insert (11, 12), which is not in contact with the data plug (24), from the receptacle (21),
Inserting the service insert (34), which is in contact with the data plug (24), in the receptacle (21),
Activating the service insert (34),
Removing the service insert (34), which is in contact with the data plug (24), from the receptacle (21),
Inserting the socket insert (11, 12), which is not in contact with the data plug (24), in the receptacle (21).

## Revendications

1. Appareil (10), destiné notamment à l'alimentation d'instruments médicaux avec des consommables, en particulier un ou plusieurs milieux fluides et/ou du courant électrique,
comprenant un boîtier dans lequel est disposé un circuit électrique (40) qui présente au moins une sortie de consommable (23) ou est relié à une telle sortie, de manière à la commander, et qui présente au moins un connecteur enfichable de données (24),
comprenant un compartiment (21) dans lequel sont disposés la sortie de consommable (23) et le connecteur enfichable de données (24),
comprenant un insert de connecteur femelle (11, 12) qui est disposé dans le compartiment (21) de manière à recouvrir le connecteur enfichable de données (24) et qui présente, côté appareil, un connecteur de consommable (32) qui est relié de façon amovible à la sortie de consommable (23), et qui présente un raccord d'instrument (14 à 19) qui est accessible de l'extérieur et est relié au connecteur de consommable (32), l'insert de connecteur femelle (11, 12) n'étant pas relié au connecteur enfichable de données (24) et ne présentant pas de raccords susceptibles d'être reliés au connecteur enfichable de données (24).

2. Appareil selon la revendication 1, dans lequel le connecteur enfichable de données (24) et la sortie de consommable (23) sont disposés à distance l'un de l'autre dans le compartiment (21).

3. Appareil selon une des revendications précédentes, dans lequel est associé à l'insert de connecteur femelle (11, 12), un dispositif de verrouillage (27a) destiné à bloquer l'insert de connecteur femelle (11, 12) dans le compartiment (21).

4. Appareil selon une des revendications précédentes, dans lequel l'insert de connecteur femelle (11, 12) présente un contour qui est adapté au compartiment (21).

5. Appareil (10) selon une des revendications précédentes, comprenant un insert de service (34) qui comporte un boîtier présentant, sur une face d'extrémité (36), un connecteur enfichable de données (37) qui est adapté au connecteur enfichable de données (24) de l'appareil (10).

6. Appareil selon la revendication 5, dans lequel l'insert de service (34) présente un contour qui est adapté au compartiment (21).

7. Appareil selon une des revendications 5 ou 6, dans lequel l'insert de service (34) est dépourvu de moyens de raccordement pour la sortie de consommable (23).

8. Appareil selon une des revendications 5 à 7, dans lequel l'insert de service (34) présente un support de stockage (34a) qui est relié au connecteur enfichable de données (37).

9. Appareil selon une des revendications 5 à 8, dans lequel l'insert de service (34) présente une interface (39) destinée à la communication avec un ordinateur.

10. Appareil selon la revendication 9, dans lequel l'interface (39) est une interface USB.

11. Appareil selon la revendication 9, dans lequel l'interface (39) est une interface réseau.

12. Appareil selon la revendication 9, dans lequel l'interface est une liaison radioélectrique.

13. Procédé d'entretien d'un appareil selon une des revendications 5 à 12, comprenant les étapes suivantes :
retrait, hors du compartiment (21), de l'insert de connecteur femelle (11, 12) qui n'est pas en contact avec le connecteur enfichable de données (24),
mise en place, dans le compartiment (21), de l'insert de service (34) qui est en contact avec le connecteur enfichable de données (24),
activation de l'insert de service (34),
retrait, hors du compartiment (21), de l'insert de service (34) en contact avec le connecteur enfichable de données (24),
mise en place, dans le compartiment (21), de l'insert de connecteur femelle (11, 12) qui n'est pas en contact avec le connecteur enfichable de données (24).
